# NOUVEAU FASCICULE DE BREVET EUROPEEN

(11) **EP 0 593 656 B3**
(45) Date de publication du présent fascicule: **07.10.2009**
(45) Mention de la délivrance du brevet: 22.01.1997
(21) Numéro de dépôt: 92915686.7
(22) Date de dépôt: 03.07.1992
(51) Int. Cl.: A61K 47/10, A61K 47/26, A61K 47/44

(54) **NOUVELLES COMPOSITIONS A BASE DE DERIVES DE LA CLASSE DES TAXANES**
Taxan-Derivate enthaltende Arzneimittel
NOVEL COMPOSITIONS BASED ON TAXANE CLASS DERIVATIVES

(30) Priorité: 08.07.1991 FR 9108527
(43) Date de publication de la demande: 27.04.1994
(73) Titulaire: Aventis Pharma S.A., 92160 Antony (FR)
(72) Inventeur: BASTART, Jean-Pierre, F-77150 Lesigny (FR); DUPECHEZ, Thierry, F-91360 Villemoisson-sur-Orge (FR); FABRE, Jean-Louis, F-75013 Paris (FR)
(74) Mandataire: Le Pennec, Magali
(86) Numéro de dépôt international: PCT/FR1992/000625
(87) Numéro de publication internationale: WO 1993/000929

(56) Documents cités:
- EP-A2- 0 118 316
- EP-B1- 0 253 738
- Journal of the National Cancer Institute, vol. 82, no. 15, 1 août 1990, E.K. ROWINSKY et al.: "Taxol: a novel investigational antimicrotubule agent", pages 1247-1259, voir page 1251, colonne de gauche: "Pharmaceutical data" (citée dans la demande)
- STN International Information Services, Base de Donnees: Chemical Abstracts, vol. 106, no. 22, 1987, (Columbus, Ohio, US), B.D. TARR et al.: "A new parenteral vehicle for the administration of some poorly water soluble anti-cancer drugs", voir abrégé no. 182581c, & J. PARENTER. SCI. TECHNOL., 41(1), 31-3, 1987, voir abrégé

## Description

La présente invention concerne une nouvelle forme pharmaceutique à base d'un agent thérapeutique ayant une activité antitumorale et antileucémique. Elle concerne plus particulièrement une nouvelle forme injectable contenant des produits de la famille des taxanes tels que notamment un des analogues du taxol ou dérivé de formule générale suivante :

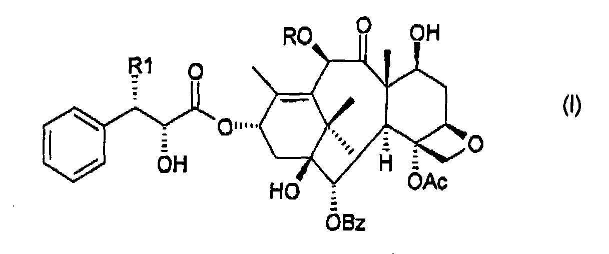

Dans la formule (I), R représente un atome d'hydrogène, le symbole R₁ représente un radical tertiobutoxycarbonylamino. Le composé est plus connu sous la dénomination de Taxotère.

Ce produit présente in vivo une activité importante sur les tumeurs malignes ce qui a permis de l'étudier dans le traitement des maladies résistantes à toutes les autres thérapies anticancéreuses.

Malheureusement ce produit présente une solubilité dans l'eau tellement faible qu'il a été nécessaire de préparer une formulation pour préparation injectable à base d'agent tensioactif et d'éthanol. L'éthanol, est le meilleur solvant qui permette de solubiliser la molécule répondant à la formule (I).

A titre d'exemple, selon la publication de Rowinsky, Lorraine, Cazenave et Donehower parue dans le Journal of the National Cancer Institute, vol. 82, No 15, pages 1247 à 1259, le 1 er Août 1990, on prépare une première solution, dite "solution mère", contenant environ 6 mg/ml de taxol dans un mélange solvant composé de :
- 50 % en volume d'éthanol
- 50 % en volume de Crémophor® EL.

Lors de l'injection, cette solution est mélangée avec un liquide de perfusion contenant du chlorure de sodium ou du dextrose. Pour obtenir un mélange stable, d'un point de vue physique comme d'un point chimique, les auteurs de cet article disent qu'il faut limiter la concentration en principe actif dans le soluté de perfusion à des concentrations d'environ 0,03 à 0,6 mg/ml (voir publication précédente page 1251, colonne 1, troisième paragraphe).

Or il est souhaitable de pouvoir injecter des doses suffisantes de principe actif, pour cela les cliniciens désirent injecter des concentrations en principe actif comprises entre environ 0,3 et 1 mg/ml dans le liquide de perfusion, au delà de ces doses apparaissent des phénomènes de chocs anaphylactiques difficiles à maîtrise dus pour l'essentiel au Cremophor® (voir la publication de Rowinsky page 1250 deuxième colonne dernier paragraphe).

Toujours selon cette publication, pour obtenir de telles concentrations (entre 0,3 et 1 mg/ml) il est nécessaire d'injecter des solutions contenant en même temps que le principe actif des concentrations en chacun des composés suivants, éthanol et surtout Crémophor®, d'environ 8 g pour 100 ml de soluté. Le traitement demandant souvent l'administration de doses élevées de principe actif et la concentration du principe actif dans la solution étant relativement faible l'injection de fort volume a pour effet de provoquer durant le traitement en plus des manifestations anaphylactiques des manifestations d'éthylisme.

Il a été découvert par la mise en oeuvre des formes pharmaceutiques de la présente invention que l'on pouvait supprimer le Crémophor® et diminuer fortement les concentrations en éthanol.

Pour cela on prépare une solution mère contenant le principe actif dans un mélange de solvants composé d'éthanol qui est le meilleur solvant biocompatible de principe actif de la classe des taxanes et d'un agent tensioactif choisi parmi les polysorbates commercialisés notamment sous la dénomination Tween.

La solution mère est préparée par dissolution du principe actif dans l'éthanol puis addition progressive de l'agent tensioactif. On peut ainsi préparer des solutions contenant 10 à 100 mg/ml de principe actif dans un mélange contenant environ 50 % d'agent tensioactif.

L'objet de la présente invention est le suivant: le Crémophor® décrit dans la publication du Journal of National Cancer Institute a été remplacé par un polysorbate. En effet, lorsque l'on utilise un soluté injectable contenant de l'éthanol et comme agent tensioactif le polysorbate 80 à la place du Crémophor®, au niveau clinique, il est apparu que les réactions anaphylactiques étaient fortement diminuées par rapport à l'utilisation du même soluté préparé avec le Crémophor®. En plus de cet avantage considérable, il est apparu de façon tout à fait étonnante que dans les flacons de solution mère, la concentration en principe actif peut atteindre 15 mg/ml. Le liquide de perfusion après dilution de ces flacons contient une quantité d'éthanol comme une quantité de tensioactif diminuée par un peu plus de deux.

Les perfusions préparées à partir des solutions mères précédentes et contenant une concentration en principe actif de, par exemple, 1 mg/ml, ce qui est une préférence, contiennent moins de 50 ml/l de polysorbate et d'éthanol, ce qui représente une diminution d'environ 40 % par rapport aux perfusions de l'art antérieur.

Ces perfusions sont stables d'un point de vue physique, c'est à dire qu'on ne voit apparaître aucun phénomène de précipitation avant environ 8 heures.

Les perfusions de Taxotère sont ensuite injectées à l'homme à un débit prédéterminé en fonction de la quantité de principe actif que l'on veut injecter. On n'observe pas avec ces solutions les phénomènes de chocs anaphylactiques que l'on observait avec les solutions de l'art antérieur.

L'invention sera plus complètement décrite à l'aide des exemples suivants qui ne doivent pas être considérés comme limitatifs de l'invention.

### EXEMPLES SELON L'INVENTION

### EXEMPLE 1

On dissout 0,450 g de Taxotère dans 15 ml d'éthanol. On complète avec le polysorbate 80 jusqu'à 30 ml pour obtenir une solution contenant 15 mg/ml de Taxotère. La stabilité physicochimique de cette solution est satisfaisante.
Cette solution après mélange avec une solution de glucose à 5 % de façon à obtenir une concentration finale de 1 mg/ml contient environ 33 ml/l de polysorbate 80 et 33 ml/l d'éthanol.
La perfusion est stable pendant plus de 21 heures. On entend par stabilité physique le fait de ne voir aucun phénomène de précipitation pendant cette période.

### EXEMPLE 2

On reproduit le même exemple avec une concentration initiale de 10 mg/ml de Taxotère les résultats sont indiqués dans le tableau 1.

### EXEMPLE COMPARATIF SELON L'ART ANTERIEUR

On dissout 0,180 g de taxol dans 15 ml d'éthanol. On complète avec du Crémophor® pour obtenir 30 ml d'une solution qui contient 6 mg/ml de taxol.
Cette solution est diluée dans le même soluté de perfusion que précédemment à raison de 1mg/ml, le soluté de perfusion contient 87,7 ml/l de Cremophor® et 87,7 ml/l d'éthanol.
La solution de perfusion est stable pendant plus de 21 heures.

### EXEMPLE 3

On dissout 65 g de Taxotere dans 2083 ml d'éthanol. On ajuste à 4147 ml par addition de 2083 ml de polysorbate 80. On homogénéise par agitation mécanique. On filtre sur un filtre de dimension de pores 0,2 µm. On obtient une solution à environ 15 mg/ml de Taxotère.

Cette solution après dilution à une teneur en Taxotère de 1mg/ml dans une poche de perfusion contenant du dextrose à 5% est stable au moins 96 heures.

**TABLEAU 1**

| Produit | Solvant | Solution mère concentration | Principe actif dans la perfusion | Tensioactif dans la perfusion | Ethanol dans la perfusion | Stabilité |
|---|---|---|---|---|---|---|
| Taxol | EtOH/Crem | 6 mg/ml | 1mg/ml | 87,7 ml/l | 87,7 ml/l | >21H |
| Taxol | EtOH/Poly | 6 mg/ml | 1 mg/ml | 83,3 ml/l | 83,3 ml/l | >21 H |
| Taxotère | EtOH/Poly | 15 mg/ml | 1 mg/ml | 33,3 ml/l | 33,3 ml/l | >21 H |
| Taxotère | EtOH/Poly | 10 mg/ml | 1 mg/ml | 50 ml/l | 50 ml/l | >21H |

## Revendications

1. Compositions à base de produits de la classe des taxanes de formule (I) 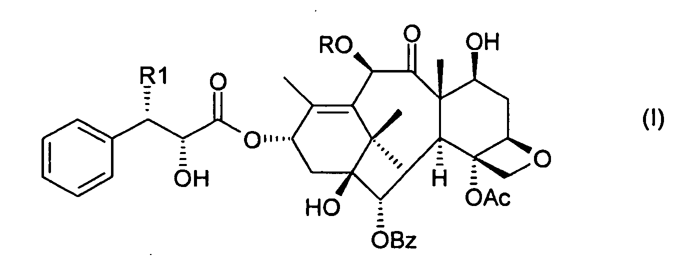 dans laquelle R représente un atome d'hydrogène et R1 un radical tertiobutoxycarbonylamino en solution dans un mélange d'éthanol et de polysorbate.

2. Compositions selon la revendication 1 **caractérisées en ce qu'**elles contiennent entre 6 et 15mg/ml de composé de formule (I) en solution dans un mélange d'éthanol et de polysorbate.

3. Perfusions **caractérisées en ce qu'**elles contiennent environ 1 mg/ml de composé de formule (I), moins de 35ml/l de polysorbate et moins de 35 ml/l d'éthanol.

## Claims

1. Compositions based on products of the taxane class of formula (I) 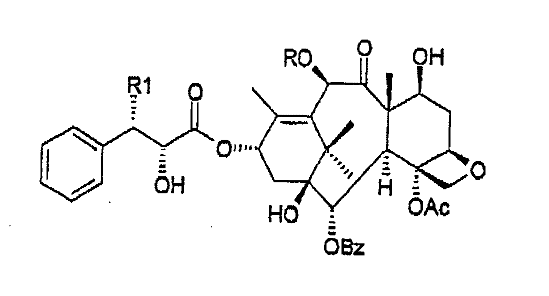 in which R represents a hydrogen atom and R1 represents a tert-butoxycarbonylamino radical, dissolved in a mixture of ethanol and polysorbate.

2. Compositions according to Claim 1, **characterized in that** they contain between 6 and 15 mg/ml of compound of formula (I), dissolved in a mixture of ethanol and polysorbate.

3. Perfusions, **characterized in that** they contain approximately 1 mg/ml of compound of formula (I), less than 35 ml/l of polysorbate and less than 35 ml/l of ethanol.

## Patentansprüche

1. Zusammensetzungen auf der Basis von Produkten der Klasse von Taxanen der Formel (I) 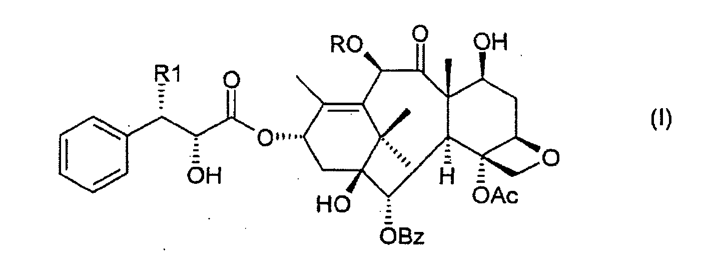 in der R ein Wasserstoffatom darstellt und R1 ein tert.-Butoxycarbonylaminorest ist, in Lösung in einer Mischung von Ethanol und Polysorbat.

2. Zusammensetzungen nach Anspruch 1, **dadurch gekennzeichnet, daß** sie zwischen 6 und 15 mg/ml Verbindung der Formel (I) in Lösung in einer Mischung von Ethanol und Polysorbat enthalten.

3. Perfusionen, **dadurch gekennzeichnet, daß** sie etwa 1 mg/ml Verbindung der Formel (I), weniger als 35 ml/l Polysorbat und weniger als 35 ml/l Ethanol enthalten.
